(19) European Patent Office

Europäisches Patentamt

European Patent Office

Office européen des brevets

(11) EP 0 874 044 A2

(12) EUROPEAN PATENT APPLICATION

(43) Date of publication:
28.10.1998 Bulletin 1998/44

(51) Int Cl.⁶: **C12N 9/08**, G01N 33/52

(21) Application number: 98303177.4

(22) Date of filing: 24.04.1998

(84) Designated Contracting States:
**AT BE CH CY DE DK ES FI FR GB GR IE IT LI LU MC NL PT SE**
Designated Extension States:
**AL LT LV MK RO SI**

(30) Priority: 25.04.1997 US 44815 P
02.04.1998 US 53923

(71) Applicant: **Ortho-Clinical Diagnostics, Inc.
Rochester, NY 14626-5101 (US)**

(72) Inventors:
• **Carlton, Dennis D.
Rochester, NY 14616 (US)**
• **Snoke, Roy E.
Webster, NY 14580 (US)**

(74) Representative: **Mercer, Christopher Paul
Carpmaels & Ransford
43, Bloomsbury Square
London WC1A 2RA (GB)**

(54) **Process for the purification of vanadium bromoperoxidase**

(57) A process for the purification of vanadium bromoperoxidase from a variety of sources is disclosed. This simple process provides highly pure enzyme for a variety of uses including in analytical assays.

EP 0 874 044 A2

## Description

Field of the Invention

The present invention relates to processes for the purification of vanadium bromoperoxidase.

Background of the Invention

There is a continuing need in medical practice and research, and in analytical and diagnostic procedures for rapid and accurate determinations of chemical and biological substances which are present in various fluids, such as biological fluids. For example, the presence of proteins, hormones, drugs, viruses, microorganisms, narcotics and steroids must be determined rapidly and accurately for effective research, diagnosis and treatment.

A wide variety of analytical methods have been developed in recent decades to detect the noted substances. The methods have become highly reliable and in some instances, suitable for automation, as well as suitable for use in kit form. Most of such methods rely on what are known in the art as "specific binding reactions" between a substance to be detected (identified herein as a "specific binding ligand" or "ligand") and a corresponding "receptor" which recognizes and reacts with the ligand specifically. Most known specific binding reactions are between immunoreactants (in "immunoassays"), such as antibodies with antigens or antibodies with haptens, but others are also known such as avidin with biotin.

In general, immunoassays can provide a qualitative and/or quantitative determination of the presence or absence (or quantity) of a specific antigen, antibody or antigen-antibody complex. In one form of immunoassay, known as a "competitive binding immunoassay", a labeled analog of the ligand to be determined is placed in competition with a fixed amount of an appropriate antibody which can react with both the ligand and the ligand analog. The label on the analog can be appropriately detected in its "free" or complexed (that is, reacted) form. Such detection will then tell the user how much ligand is in the sample being tested.

In an alternative immunoassay format known as a "sandwich" immunoassay or immunometric assay, the ligand is contacted with two or more receptor molecules which bind to the ligand at different epitopic sites. One receptor is appropriately labeled and the other is either immobilized on a solid substrate, or is capable of being immobilized thereon. The amount of ligand is directly proportional to the amount of bound complex among the ligand and the two receptors.

In the immunoassays using peroxidase as the label, the stability of the peroxidase is highly important since any change in its concentration critically affects assay sensitivity. In the assays described in U.S. Patent No. 5,372,932 to Friedman et al., 4'-hydroxy or 4'-alkoxyarylacetamides are used as agents to enhance the stability of the enzyme or enzyme label in a dry analytical element. Although these agents have resulted in improved stability, it has been observed that the stability of the peroxidase label is still less than desirable in dry analytical elements.

Recently, Butler and Walker have described a family of vanadium bromoperoxidases (VBrPO) extracted from aquatic and marine algae and some from terrestrial lichens and fungi that, in the presence of hydrogen peroxide and bromide anion, catalytically produce activated bromine species which are potent oxidants. (See, Butler et al., Chem. Revs., 93, pp. 1937-1944, 1993 and references cited therein).

One of the problems with most haloperoxidases is that their pH optima are generally in the lower pH range, i.e., 3-5. This presents a problem with analytical elements which depend upon a peroxy anion; peroxides typically have a pKa of about 11.5. The vanadium bromoperoxidases work effectively in a pH range of about 6-10.

The problems identified above in regard to analytical elements and immunoassays have been overcome by utilizing an analytical element which comprises, in at least one of its zones, a vanadium bromoperoxidase-labeled immunoreactant.

Vanadium bromoperoxidase (VBrPO) has a number of chemical properties that make it an excellent signal-generator candidate for use in diagnostic assays. However, difficulties in purifying VBrPO from its native source (seaweed) have led to limited availability and precluded in-depth evaluation of the enzyme.

Extraction of haloperoxidases from natural sources such as seaweed is difficult because of the large amount of contaminating carbohydrate (e.g. algin), phenolics, pigments, and other contaminants (e.g. enzyme inhibitors and modfiers). Vanadium bromoperoxidase (V-BrPO) is one of several haloperoxidases found in seaweeds (e.g. A. nodosum; Wever, R., de Boer, E., Plat, H., & Krenn, B.E. (1987) Vanadium - an element involved in the biosynthesis of halogenated compounds and nitrogen fixation. FEBS Letters 216(1), 1-3.)). In the past, V-BRPO has been extracted by a low yield process that generated large volumes of dilute impure enzyme that required subsequent cumbersome processing to reduce volumes (e.g. ultrafiltration and ammonium sulfate precipitation) and remove viscous carbohydrates (e.g. calcium chloride or ethanol precipitation), dark pigments, phenolics and particulates before conventional purification techniques (e.g. liquid column chromatography) could be employed (Wever, R. Plat, H., and de Boer, E. (1985) Isolation procedure and some properties of the bromoperoxidase from the seaweed Ascophyllum nodosum. Biochim. Biophys Acta 830, 181-186). Indeed, Wever et al.(1987), supra, reported the inefficiency of the isolation process, which required repeated extraction of the same material several times to effect a reasonable yield of V-BrPO. The same group reported the failure of detergents, ionic strength or polarity modifications, and assorted mechanical disruptions of the seaweed to increase yields.

The use of two-phase aqueous partition systems (TPAS) for the isolation and purification of bio-products has been previously reviewed (Mattiasson, B. & Kaul, R. (1986) Use of Aqueous Two-Phase Systems for Recovery and Purification in Biotechnology. Separation, RecoveEy, and Purification in Biotechnoloizy, Recent Advances and Mathematical Modeling, J.A. Asenjo and J. Hyong eds. ACS, Washington; Abbott, N.L. & Hyatton, T.A.(1988) Liquid-Liquid Extraction for Protein Separations; Chemical Engineering Progress 84(8),31-41). Early attempts often proved uneconomical, employing expensive polymers (e.g. dextran) or affinity-ligands to effect the separation or to maintain the two-phase system. The latter also required a subsequent chromatography step to remove large quantities of polymer from the purified protein.

Eventually the availability of inexpensive reagents (e.g. phosphates) for phase stabilization or polymer removal facilitated commercialization of TPAS(Veide, A., Smeds, A-L., & Enfors, S.O. (1983) A Process for Large-Scale Isolation of P-Galactosidase from E. coli in an Aqueous Two-Phase System. Biotechnology and Bioengineering XXV, 1789-1800; Hustedt, H., Kroner, K.H., Menge, U., & Kula,M-R.(1985) Protein recovery using two-phase systems. Trends in Biotechnology 3(6), 139-144; Dove, G.B. & Mitra, G. (1986) Recovery of Proteins from Polyethylene Glycol-Water Solution by Salt Partition. Separation, Recovery, and Purification in Biotechnology. Recent Advances and Mathematical Modeling, J.A. Asenio and J. Hyong eds. ACS, Washington; Miranda, M.V., Femandez, L., & Cascone, 0. (1995) Horseradish Peroxidase Extraction and Purification by Aqueous Two-Phase Partition. Applied Biochemistry and Biotechnology 53, 147-154; Femandez Lahore, H.M., Miranda, M.V., Fraile, E.R., de Jimenex Bonino, M.J.B., & Cascone, 0. (1995) Process Biochemistry 30(7),615-621).

## Summary of the Invention

This invention provides improved purification procedures for haloperoxidases that use two-phase aqueous partition chromatography for isolation from either natural sources (e.g. seaweed) or from other sources (e.g. fermentations, cell-cultures, or genetically-manipulated cell lines).

The two-phase aqueous chromatography purification process for VBrPO of the present invention has the advantages of economy, simplicity of equipment, ease of scale-up, and employs mild isolation conditions that contribute to the enzyme's chemical and physical integrity and stability. The process of the present invention produces a concentrated, purified isolate that facilitates down-stream processing for applications requiring high-purity material.

The present invention provides a highly sensitive and stable composition for use in dry analytical elements. The element of the instant invention is useful for determining a wide variety of specific binding ligands, but it is particularly useful for the determination of ligands at low concentrations. The advantages of enzyme stability and enhanced detection are achieved by incorporating a vanadium bromoperoxidase-labeled immunoreactant into the element.

This invention provides a VBrPO purification scheme and a partition chromatography step that eliminates many of the difficulties peculiar to plant and seaweed extractions. Issues relevant to raw material and enzyme handling, chromatographic optimization, process reproducibility, scale-up, and product quality are addressed in the present invention. The procedure of the present invention is efficient, economical, and scaleable. This methodology to purify bromoperoxidase from recombinant sources is also included in the process of the present invention.

## Detailed Description of the Invention

TPAS's provide for a simple, economical, high-yield extraction of VBrPO from natural sources (e.g. seaweed) or other sources while simultaneously concentrating and purifying the haloperoxidase. Most cell debris and the majority of phenolics, carbohydrates, pigments, contaminating proteins and enzyme inhibitors are removed by this technique. In addition, the resulting product is stable to storage under a variety of conditions, (e.g. room temperature, freezing, and lyophilization) and can be processed readily for downstream purification procedures (e.g. column chromatography, ultrafiltration). TPAS's are also applicable to the purification of haloperoxidases from fermentation or cell-culture derived products and may be easily incorporated into automated formats (e.g. counter current chromatography) that provide extremely high resolution without the limitations of traditional or high pressure column chromatography.

In the present invention, a TPAS using a polymer, including but not limited to, PEG 8,000, and a salt, including but not limited to, potassium phosphate, is successfully applied to the purification of a haloperoxidase. We have demonstrated the mobility of V-BrPO between phases under conditions of varied pH, salt concentration, and PEG concentration and have utilized this mobility to isolate and purify VBrPO from A. nodosum.

For isolation from natural sources (including but not limited to seaweed), the starting material is homogenized (e. g. blender or Polytron TM ) and in the same or subsequent step mixed with the TPAS components (e.g. salt and PEG) to effect the extraction. The phases are then allowed to separate, a step greatly enhanced by centrifugation. Isolation of the individual phases (e.g. PEG, salt, solids) or their interface completes the process. For example, after the homogenization and centrifugation of A. nodosum, the majority of V-BrPO activity is found at the polymer-salt interface. Additional activity may be retrieved by reprocessing the other phases, but generally phenolics, pigments, inhibitors and contaminating proteins are discarded with the polymer phase. The majority of extracted carbohydrates (e.g. algins) are discarded with the salt phase. Reprocessing of the solids (pellet) results in the greatest retrieval of residual V-BRPO activity. Typically, greater than 50% of the detectable V-BRPO activity is isolated in the first extraction, with an additional 20% recovered in one reextraction of the solids.

Isolation of haloperoxidase from other sources is similar to the process for natural sources. In systems containing haloperoxidase the enzyme may be much more mobile between phases and yield a more highly purified product. Typically, conditions are first adjusted to partition the haloperoxidase (e.g. V-BrPO) into the polymer phase so that hydrophilic contaminants may be discarded with the salt phase. Upon mixing with a fresh salt phase, the haloperoxidase is partitioned back into the salt phase so that most polymer and remaining contaminants may be discarded with the polymer phase.

Alternatively, a TPAS may be employed in other formats, including but not limited to counter-current chromatography. The advantages of the latter are its high selectivity and degree of automation.

In summary, the two immiscible aqueous phases may be formed by indigenous or added polymers, salts, or carbohydrates. Selective partitioning is achieved by manipulating the characteristics of the phase components, which include but are not limited to, polymer type (including ligands), molecular weight, and concentration; salt type and concentration; pH; and temperature.

We have found that VBrPO purified according to the process of the present invention has greatly enhanced purity and is stable to storage under a variety of conditions, including but not limited to, frozen, refrigerated, ambient temperature, and lyophihzed or freeze-dried.

As used herein, "vanadium bromoperoxidase" is meant to be any vanadium haloperoxidative substance (enzymatic or otherwise) which catalyzes the oxidation of a substance such as a dye or other signal generating compound, to produce an appropriate signal. Specific examples of proteinaceous vanadium bromoperoxidase sources that may be employed in the present invention can be found in Butler et al., Chem. Revs., 93, pp. 1937-1944, (1993) and include, but are not limited to, those obtained from Ascophyllum nodosum, Ceramirum rubrum, Laminaria saccharina, Fucus distichus, Corallina pilulifera, Corallina officinalis, Macrocystis pyrifera and the like. Of these proteinaceous vanadium bromoperoxidase sources, Ascophyllum nodosum is particularly preferred.

Vanadium bromoperoxidase (VBrPO) is one of several haloperoxidases found in seaweed (e.g. *A. nodosum)* and is of great interest as a potential signal generating reagent for *in vitro* diagnostic products [Wever, R., de Boer, E., Plat, H., & Krenn, B.E. (1987). Vanadium - an element involved in the biosynthesis of halogenated compounds and nitrogen fixation. *FEBS Letters* 216(1), 1-3. Groulx, S.F., Friedman, A.E., and Butler, A. (1995). Patent pending, Use of Vanadium Bromoperoxidase as a Signal Generating Enzyme for Chemiluminescent Systems: Test Kits and Analytical Methods. Groulx, S.F., Kopcienski, M., Friedman, A.E., Kissel, T.R. (1995). Patent pending, Analytical Element and Method for the Determination of a Specific Binding Ligand using a Vanadium Bromoperoxidase as a Signal-Generating Enzyme.] In general, extraction of enzymes from seaweed is problematic because of the deluge of carbohydrates, phenolics, pigments and nucleic acids released during homogenization. Historically VBrPO has been extracted by a series of labor-intensive, low resolution steps that generate large volumes of dilute but viscous homogenate. Extensive processing is required to reduce volume, viscosity and color before conventional chromatographic procedures can be employed to substantially improve purity and reduce the potential for irreversible inhibition and chemical modification by reactive contaminants [Loomis, W.D. (1974). Overcoming Problems of Phenolics and Quinones in the Isolation of Plant Enzymes and Organelles. In Methods in Enzymology XXXI. Biomembranes Part A, eds. Fleischer, S. & Packer L. (Academic Press, N.Y.) pp 54-55]. Wever *et al* described the inefficiency of VBrPO extraction from seaweed, including: the necessity of multiple reextractions of the tissue in large volumes of buffer, the ineffectiveness of detergent, ionic strength and polarity modifications to improve yield, and finally the failure of mechanical homogenization to more efficiently release bound VBrPO [Wever, R. Plat, H., and de Boer, E. (1985). Isolation procedure and some properties of the bromoperoxidase from the seaweed *Ascophyllum nodosum. Biochim. Biophys Acta* 830, 181-186].

The use of two-phase aqueous partition systems (TPAS) for the isolation and purification of bio-products has been previously reviewed [Mattiasson, B. & Kaul, R. (1986). Use of Aqueous Two-Phase Systems for Recovery and Purification in Biotechnology. Separation, Recovery, and Purification in Biotechnology. Recent Advances and Mathematical Modeling, J.A. Asenjo and J. Hyong eds. ACS, Washington. Abbott, N.L. & Hyatton, T.A. (1988). Liquid-Liquid Extraction for Protein Separations. *Chemical Engineering Progress* 84(8), 31-41.] It is a particularly attractive technique for manufacturing because it avoids the shortcomings of traditional column technologies in that it is nondenaturing, tolerant of a variety of sample types, requires only simple instrumentation, yet is highly selective. Early TPAS industrial applications frequently proved uneconomical, employing expensive polymers or affinity-ligands and requiring a subsequent column chromatography step to isolate the purified product from the polymer. The eventual discovery of effective nonpolymeric reagents for phase stabilization provided an alternative avenue for polymer removal and facilitated commercialization of two-phase aqueous systems [Veide, A., Smeds, A-L., & Enfors, S.O. (1983). A Process for Large-Scale Isolation of β-Galactosidase from E. coli in an Aqueous Two-Phase System. *Biotechnology and Bioengineering* XXV, 1789-1800. Hustedt, H., Kroner, K.H., Menge, U., & Kula, M-R. (1985). Protein recovery using two-phase systems. *Trends in Biotechnology* 3(6), 139-144. Miranda, M.V., Femandez, L., & Cascone, O. (1995). Horseradish Peroxidase Extraction and Purification by Aqueous Two-Phase Partition. *Applied Biochemistry and Biotechnology* 53, 147-154]. Cell debris and the majority of phenolics, nucleic acids, carbohydrates, pigments, contaminating proteins and enzyme inhibitors are removed by the process of the present invention.

The following examples are given to illustrate the scope of this invention. Because these examples are given for illustrative purposes only, the invention embodied therein is not intended to be limited thereto.

Except where noted, all reagents and equipment were obtained from commercial sources.

## EXAMPLE 1

Two-phase purification of Vanadium Bromoperoxidase

*A. nodosum* was collected from the English shoreline. Trizma-base, MOPS, dibasic potassium phosphate, monobasic potassium phosphate, polyethylene glycol, potassium bromide, phenol red, hydrogen peroxide and ammonium metavanadate were purchased from Sigma. Ultra-pure ammonium sulfate was obtained from ICN. High purity urea was purchased from Sigma (Ultra-) or Pierce (sequanal-grade). Nanopure$^{TM}$ water was used in all instances.

Traditional VBrPO extraction protocol. A modified version of the methods of Wever et al and Butler was initially used to prepare VBrPO extracts from *A. nodosum* for study[6] [Butler, A. Personal communication]. Raw seaweed was thawed, washed, and if necessary the pods and stems sorted prior to refreezing for storage. Typically, our lab used ~60g of pods per extraction. The pods were frozen in liquid nitrogen and pulverized using a mortar and pestle to maximize surface area. VBrPO was extracted using 0.1-0.4L 0.2M tris-sulfate pH 8.3 in a chilled blender. Extract and solids were separated by centrifugation, with the solids being reprocessed with fresh buffer an additional 3-9 cycles. For evaluation of individual cycle homogenates, supernatants were not pooled. Otherwise, the most active supernatants were pooled, then treated with 0.1M calcium chloride and centrifuged. The resulting pellets were discarded and the clarified extract brought to 80% saturation with solid ammonium sulfate (AS) under refrigerated conditions. After >12 hours of stirring, precipitated and solublized fractions were collected by centrifugation for further study.

Air drying *A. nodosum.* Whole *A. nodosum* was surface sterilized for about 2 minutes using 5-10% bleach. After rinsing with water, the seaweed was placed on screens and air dried at 37°C for 48-72 hours. A dehumidifier maintained the humidity at ≤45% to prevent mold growth. Dried seaweed has been stored for at least 6 months in opaque plastic bags at ambient temperature without loss of VBrPO activity.

VBrPO isolation using a TPAS. Dried *A. nodosum* was powdered by blending for 15-30 seconds or wet *A. nodosum* by pulverizing in liquid nitrogen with a mortar and pestle. The powder was typically separated into 20g portions for individual extraction in a 2L Hamilton blender with 0.6L buffered dibasic potassium phosphate (KP$_i$) pH 8.3, at 3-8°C for 4-5 minutes [The potassium phosphate was typically buffered with 50mM tris-sulfate to maintain alkaline conditions during the isolation procedure (pH 8.3). Sulfuric acid was used for titrations.]. Then 0.36L 33.3% w/w polyethylene glycol (PEG) 8000 and 0.04L water were added and blending resumed for 10-12 minutes. [Blending was typically broken up into multiple short steps to avoid warming the sample, e.g. 1 min blend, 2 min cooling, repeat cycle 9-11 times. Blend intervals up to 5 minutes have proven successful.] The homogenate was centrifuged in 0.5L centrifuge bottles for at least 45-60 minutes at ~12,000rcf, ~8°C, to effect the phase separation. The PEG (top) and KP$_i$ salt (bottom) phases were decanted and the mat material at the PEG-salt interface collected. Frequently, an optional second centrifugation step succeeded the first. Mat was collected into 0.05L centrifuge tubes, balanced using salt phase and then centrifuged for at least 20 minutes at ~27,000rcf, ~8°C. Isolated mat was frozen and typically lyophilized for storage at ambient temperature in amber glass bottles.

VBrPO Activity. VBrPO activity was measured on a Beckman DU-70 spectrophotometer, generally after preincubation of the enzyme in 0.1-1.0mM ammonium metavanadate. Activity was expressed as micromoles of phenol red converted per minute (U), measured as $\Delta A592/min$ in 35 μM phenol red (PR), 0.1M potassium bromide, and 0.1M monobasic potassium phosphate pH 6.5 at 25°C. [Manual selection of a linear portion of the A592 was necessary due to an initial lag phase. The addition of enzyme was adjusted to keep the A592 below 1.3 over the duration of the 10 minute assay. (Extinction coefficient = 67.4.) ] Reactions were initiated with 1mm hydrogen peroxide.

Partition coefficient ($K_{part}$). Assorted two phase aqueous systems were generated and partially purified VBrPO spiked into the mixtures. The relative enzyme concentration (c) in each layer was measured as VBrPO activity and the partition coefficient ($K_{part}$) calculated as described below.

$$K_{part} = c_{top}/c_{bottom}$$

Mat activity and lyophilized mat VBrPO stability measurements. VBrPO activity in fresh mat or pellet was determined by suspending 0.1 g in 1 ml 50mM tris-sulfate pH 8.3 +/- 1.0mM ammonium vanadate. The mixture was vortexed and activity measured as described above after an additional 5-fold dilution in buffer. Wide-mouth pipette tips were necessary for accurate dispensing of aliquots. VBrPO stability in lyophilized mat was determined by rehydration in tris-sulfate buffer or tris-sulfate buffered 2M urea, followed by incubation +/- 1.0mM ammonium vanadate.

Chromatographic analyses. High performance size exclusion (HPSEC) chromatography was performed on a Hewlett Packard 1050 liquid chromatograph using Toso Haas TSK columns at ambient temperature. Buffers were degassed using helium sparging. All samples were filtered to <0.5 μm prior to injection.

Evaluation of a conventional VBrPO purification procedure. In general the extraction and purification techniques chosen to routinely process kilograms of seaweed at a time proved quite inefficient. In addition to the unique challenges afforded plant tissue extractions, evaluation of the methods of Wever et al and Butler (Table 1) also revealed difficulties peculiar to A. nodosum and VBrPO[6,12]. For example, VBrPO extractions were typically preceded by manual sorting of pods from stems in order to obtain one kilogram of VBrPO-enhanced starting material. To eliminate the labor and material waste imposed by manual sorting, we evaluated both stem and pod extractions. Yields were found to be similar (data not shown), with stems releasing VBrPO more quickly. However, while VBrPO was successfully purified during an early evaluation of phenyl Sepharose[TM], the same column did not bind VBrPO from a stem extract.

Table 1.

| VBrPO purification procedures of Wever et al and Butler[6,12]. | |
| --- | --- |
| Step | Procedure |
| 1 | Sort pods from stems |
| 2 | Homogenization/centrifugation, ~6-8 cycles |
| 3 | Precipitation (CaCl$_2$), centrifugation |
| 4 | Hollow fiber ultrafiltration (10kDa MWCO) |
| 5 | Precipitation (80% ammonium sulfate), centrifugation |
| 6 | Homogenization (60% ethanol), centrifugation |
| 7 | Precipitation (90% ethanol), centrifugation |
| 8 | Anion exchange chromatography |
| 9 | Size-exclusion chromatography |
| 10 | Ultrafiltration, dialysis |

Likewise, stem extracts performed poorly during salting out experiments, requiring extended incubation in 100% saturated ammonium sulfate. Subsequent HPLC analyses showed the distribution of contaminants released by early versus late blender cycles to be quite different. Since stem and pod extracts constitute different pools of the blender homogenates, it appeared that either stem-derived or early-release contaminants may have been responsible for the pod sorting legacy.

The choice of 0.2M tris for extractions dictated the use of large volumes of buffer and exhaustive mechanical disruption to achieve an

Table 2.

| Effects of buffer volume, 5%w/v PVPP, and 0.1mM vanadium on individual yields of four separate but consecutive blending cycles (A-D). Extract #1, 150ml fresh buffer/cycle; Extract #2, 500ml fresh buffer/cycle. Extractions started with 20g wet whole plant. | | | | | |
| --- | --- | --- | --- | --- | --- |
| Phenol Red Activity (U) | Untreated raw extracts | | PVPP treatment, no added vanadium | | 30min V incubatio raw extra |
| Blending cycle: | Extract #1(U) | Extract #2(U) | Extract #1(U) | Extract #2(U) | Extract #1(U) |
| A | 1.7 | 4.2 | 2.1 | 10.8 | 4.7 |
| B | 4.0 | 13.0 | 18.8 | 23.2 | 12.4 |
| C | 3.4 | 7.4 | ND | ND | 12.2 |
| D | 10.4 | 10.4 | ND | ND | 5.2 |
| Total | 19.5 | 35 | (20.9) | (34) | 34.5 |

acceptable release of VBrPO from the seaweed (Table 2). Using 0.15L buffer per cycle, we found that at least nine homogenization cycles were required for a thorough extraction of 20g wet *A. nodosum.*

The competition between reactivation of apo-VBrPO and the formation of inactive or inhibited VBrPO species greatly impeded simple tracking and yield determinations. Indeed, outside reports commonly postponed reporting extraction yields until downstream processing had already been initiated, or they recorded increasing yields as the purification progressed. Table 2 data suggested that scavengers (i.e. PVPP) could offer significant protection during later cycles (B), but the tendency for alkaline conditions to impede the effectiveness of these agents suggests one possible mechanism for limited PVPP utility in the initial blend cycle (A). VBrPO sensitivity to thiol reagents precluded the use of antioxidants such as dithiothreitol.

The calcium chloride step used by Butler to remove alginates reduced viscosity somewhat, but reduced overall yield and necessitated another large-scale centrifugation step (Table 3)[12]. The resulting supernatant was still slow to concentrate by ultrafiltration, again lengthening the exposure of VBrPO to potentially destructive species. In addition, *A. nodosum* is unique in its production of soluble algins that defy calcium chloride treatments, necessitating at least one downstream ethanol precipitation step (Table 1) [Muzzarell, R.A.A. (1973). Chapter 2: *Alginic Acid.* In Natural Chelating Polymers. Alginic Acid, Chiten and Chitosan. Pergamon Press, N.Y. Myklestad, S. & Haug, A. (1965). In Proc. 5th Int. Symp. Halifax, Nova Scotia, Young & McLachlan eds. Pergamon Press.]

Table 3.

| Effects of CaCl$_2$ precipitation on VBrPO yields (U) in individual homogenates from four blending cycles (A-D) [Samples were incubated in 0.1mM ammonium vanadate prior to assay. ] Extract #1, 150ml fresh buffer/cycle; Extract #2, 500ml fresh buffer/cycle. Extractions started with 20g wet whole plant. | | | | |
|---|---|---|---|---|
| Phenol Red Activity | Raw extracts | | CaCl$_2$ treated extracts | |
| Blending cycle: | Extra ct #1 (U) | Extra ct #2 (U) | Extra ct #1 (U) | Extract #2(U) |
| A | 4.7 | 30.4 | 7.0 | 38.5 |
| B | 12.4 | 26.5 | 5.2 | 20.7 |
| C | 12.2 | 25.2 | 4.8 | 5.5 |
| D | 5.2 | 14.0 | 2.1 | 0.9 |

Ammonium sulfate fractionation (Table 1, step 5) produced large quantities of precipitate and was found to be moderately effective at reducing pigment levels. However, a significant amount of VBrPO and 80% of the protein remaining after the calcium chloride treatment did not precipitate. BioRad and Pierce BCA[TM] protein assays showed the precipitate to be almost entirely nonproteinaceous, probably carbohydrates [Fang, G., Hammar, S., & Grumet, R. (1992). A Quick and Inexpensive Method for Removing Polysaccharides from Plant Genomic DNA. *BioTechniques,* v13(1), 52-56. ] Of the VBrPO and protein that had precipitated, most remained bound to solids even after resuspension. Subsequent ethanol precipitation and centrifugation steps (Table 1, steps 6-7) imposed an unnecessary safety risk and therefore were not performed on a preparative-scale in our labs. However, small-scale testing suggested VBrPO was lost during the 60% ethanol cut unless an exhaustive effort was made to remove residual AS beforehand.

Development of a TPAS model. The persistent slow-release of VBrPO from *A. nodosum* despite extensive mechanical homogenization, together with the dependency upon large buffer volumes, suggested that conditions had not been optimized to favor partitioning of the enzyme into the extraction medium. It was believed that a liquid-liquid extraction system would offer the enzyme a more favorable environment, increasing both extraction efficiency and VBrPO purity in a single step. Two-phase aqueous partition chromatography was selected for its mild conditions and environmental compatibility.

Typically, protein purification by TPAS is a four step procedure:

(1) generation of two phases under high $K_{part}$ conditions,
(2) isolation of the target-rich PEG phase,
(3) regeneration of a TPAS under low $K_{part}$ conditions, and finally
(4) isolation of the PEG-free, target-rich salt phase.

Therefore it was indeed necessary to chose a system that would allow VBrPO mobility between phases in order to facilitate bulk removal of contaminants. Initial adjustment of parameters was based upon the literature (Table 4) [Femandez Lahore, H.M., Maranda, M.V., Fraile, ER, de Jimenex Bonino, M.J.B., & Cascone, O. (1995). Partition Behavior and Purification of a Mucor bacilliformis Acid Protease in Aqueous Two-Phase Systems. *Process Biochemistry* 30(7), 615-621. ] Pigments have been reported to always favor the PEG phase, as phenolics were anticipated to do, while carbohydrates favor the salt phase. Since proteins rarely partition out of PEG 600 or into PEG 20,000, PEGs 1450, 3450, and 8000 were chosen for experimental testing.

### Table 4. Parameters effecting separations [$K_{part}$] in a TPAS.

| Chemical nature of primary polymer (PEG) and secondary agent (KPi). |
| --- |
| Concentration of primary polymer, secondary agent |
| Additives (e.g. NaCl, detergent, ligands). |
| pH |
| Temperature |
| Chemical nature of VBrPO |

Although other salts (e.g. sulfates) may eventually prove to be more applicable to the isolation of VBrPO from contrived sources, dibasic potassium phosphate was selected for seaweed extractions because it is the most commonly reported TPAS salt and has an alkaline starting pH. Dove and Mitra have reported that alkaline conditions facilitate phase separation [Dove, G.B. & Mitra, G. (1986). Recovery of Proteins from Polyethylene Glycol-Water Solution by Salt Partition. In Separation, Recovery, and Purification in Biotechnology. Recent Advances and Mathematical Modeling, J.A. Asenjo and J. Hyong eds. ACS, Washington. ]. Because $K_{part}$ for proteins increases additively with increasing pH and salt concentration, generating the initial TPAS was straight forward. The challenge was to simultaneously develop a complimentary low $K_{part}$ TPAS that was stable under conditions of reduced pH or salt.

Stability data from the first experiments are shown in Tables 5 and 6. Despite the critical discovery that TPAS

Table 5.

| Effects of salt concentration on a TPAS at pH>9. | | | |
| --- | --- | --- | --- |
| PEG 3450 (%w/v) | $Kp_i$ (M) | pH | Results (+/-) |
| 13.3 | 1.50 | 9.6 | + |
| 13.3 | 1.20 | 9.2 | + |
| 13.3 | 0.90 | 9.1 | + |
| 13.3 | 0.60 | 9.1 | + |
| 13.3 | 0.30 | 9.1 | - |
| 13.3 | 0.15 | 9.1 | - |

Table 6.

| Effects of PEG 3450 concentration on a TPAS at pH>9. | | | |
| --- | --- | --- | --- |
| PEG 3450 (% W/V) | $KP_i$ (M) | pH | Results (+/-) |
| 10.0 | 0.9 | 9.1 | + |
| 6.7 | 0.9 | 9.1 | + |
| 3.3 | 0.9 | 9.0 | - |
| +/- Indicates the presence or absence of a TPAS, respectively. | | | |

treated VBrPO required ten fold more vanadate for reactivation than enzyme purified by conventional means, the system displayed an unusually high $K_{part,}$ ~500. Since the primary goal was VBrPO mobility between phases, the system would have had to be extraordinarily tolerant of large reductions in salt concentration and pH to significantly reduce $K_{part}$. PVPP was found to partition solely into the PEG phase, suggesting a means for early absorption and removal of phenolics.

Table 7 shows the results of varied pH and PEG molecular weight on phase formation. A PEG concentration of ~12% w/v was maintained since it offered sufficient phase stabilization with decreasing salt and pH. VBrPO spiking experiments using PEG 1500 and 3450 systems did not suggest that a reduction of $K_{part}$ to less than single digits was possible. However, PEG 8000 offered a $K_{part}$ of only 0.4 at pH 8 when salt was reduced to just 0.9M Kpi. In addition to providing a robust TPAS, it offered a particularly exciting purification opportunity since many proteins do not partition into such high molecular weight PEGs.

Table 7. Effect of pH and PEG molecular weight on a TPAS at 4°C.

| PEG 1500 | | $KP_i$ (M) | TPAS (+/-) | PEG 3450 | | $KP_i$ (M) | TPAS (+/-) | PEG 8000 | | $KP_i$ (M) | TPAS (+/-) |
|---|---|---|---|---|---|---|---|---|---|---|---|
| 12% | | 1.65 | + | 12% | | 1.65 | + | 12% | | 1.65 | + |
| 12 | | 1.50 | + | 12 | | 1.50 | + | 12 | | 1.50 | + |
| 12 | | 1.20 | + | 12 | | 1.20 | + | 12 | | 1.20 | + |
| 12 | | 0.90 | + | 12 | | 0.90 | + | 12 | | 0.90 | + |
| 12 | | 1.65 | + | 12 | | 1.65 | + | 12 | | 1.65 | + |
| 12 | | 1.50 | + | 12 | | 1.50 | + | 12 | | 1.50 | + |
| 12 | | 1.20 | + | 12 | | 1.20 | + | 12 | | 1.20 | + |
| 12 | | 0.90 | + | 12 | | 0.90 | + | 12 | | 0.90 | + |
| 12 | | 1.65 | + | 12 | | 1.65 | R | 12 | | 1.65 | + |
| 12 | | 1.50 | + | 12 | | 1.50 | + | 12 | | 1.50 | + |
| 12 | | 1.20 | + | 12 | | 1.20 | + | 12 | | 1.20 | + |
| 12 | | 0.90 | - | 12 | | 0.90 | - | 12 | | 0.90 | + |
| 12 | | 1.65 | R | 12 | | 1.65 | R | 12 | | 1.65 | R |
| 12 | | 1.50 | R | 12 | | 1.50 | + | 12 | | 1.50 | + |
| 12 | | 1.20 | R | 12 | | 1.20 | + | 12 | | 1.20 | + |
| 12 | | 0.90 | R | 12 | | 0.90 | - | 12 | | 0.90 | - |

(+/-) Presence or absence of two phases. (R) Salt recrystallized.

TPAS Fractionation of *A. nodosum.* The PEG 8000 model was tested using wet *A. nodosum* which had been liquid nitrogen frozen and pulverized with a mortar and pestle. Extraction and centrifugation yielded four fractions: green pelleted solids, a light-colored salt layer, a dark-green solid interface layer (mat), and a greenish-brown PEG layer. Little VBrPO activity was detected in the salt or PEG layers, despite several reactivation attempts. Activity assays of the pelleted solids from each blending cycle confirmed that VBrPO activity levels had decreased as expected. Spiking experiments performed on the extracts eventually confirmed that PEG-bound VBrPO was irreversibly inhibited by an unknown seaweed component. VBrPO activity was eventually found to have been concentrated in the intermediate layer, removed from the bulk of the phenolics, carbohydrates, solids, and brown pigments.

Microscopic examination of the mat showed it to be composed primarily of membrane fragments and small green spheres that were similar in appearance to chloroplasts. Vigorous physical homogenization of the isolated mat three times in fresh 0.2M tris-sulfate pH 8.3 appeared to solublize only 15% of the total mat activity. Since the enzyme was very tightly bound in the mat, loss of VBrPO to the PEG layer was thereafter assumed to be minimal. Lyophilization of the mat produced a concentrated product that was convenient for handling and ambient temperature storage. Subsequent purification development efforts focused largely on the lyophilized mat.

Follow-up isolation experiments revealed that mat formation was consistent only under specific conditions. For example pH 9.3 yielded only an ill-defined intermediate layer. No mat formed in a system composed of 18% PEG 8000 + 0.65M $KP_i$ pH 8.3, in which VBrPO activity was confined to the salt layer. Addition of PVP or PVPP to the system to absorb phenolics provided no benefit. Indeed, the latter interfered with mat recovery after centrifugation. Regardless, speed limitations incurred by our centrifuge eventually prompted a second centrifugation step to reduce solids (e.g. PEG). Mat fluidity was reduced while recovery was greatly facilitated.

Drying various *A. nodosum* samples in a vacuum oven revealed significant variability in moisture content, even between wet samples. English harvests #1 and #2 contained 60% and 80% water, respectively, suggesting that the apparent higher activity of harvest #1 material could be artifactual. Changes in sample preparation were needed to ease seaweed storage requirements, improve sample uniformity, and increase process efficiency. Surface sterilizing and air drying reduced the stench and eliminated the need for refrigerated or frozen storage. But, more importantly it also allowed substitution of a blender for the liquid-nitrogen cooled mortar and pestle initially used to pulverize the seaweed. Table 8 shows a comparison of two crude English samples, one air dried English sample and sun dried or processed *A. nodosum* from Nova Scotia. Wet samples were frozen in liquid nitrogen and hand ground, while dried samples were processed in a blender. The dried English sample suffered a 21% loss in VBrPO activity upon air drying, but this was more than justified by the gain in throughput, safety, and

Table 8.

| Comparison of different *A. nodosum* starting materials under equivalent TPAS conditions (1.2M $KP_i$). Wet plant masses were adjusted to their dry weight equivalent to allow direct yield comparisons with dried plant preps. | | | |
|---|---|---|---|
| Sample | Lyophilized Mat / Dry Seaweed (g/g) | Mat Activity / Dry Seaweed (U/g) | VBrPO Yield (Normalized) |
| English harvest #1, wet | 2.33 | 11.2 | 0.81 |
| English harvest #2, wet | 0.86 | 13.9 | 1.00 |
| English harvest #2, dried | 0.78 | 11.0 | 0.79 |
| Nova Scotia, wet | 1.3 | 4.0 | 0.29 |
| Nova Scotia, sun-dried | 0.9 | 3.0 | 0.22 |
| Nova Scotia, dry granules | 0.84 | 6.0 | 0.43 |
| Nova Scotia, dry powder | 0.44 | 1.5 | 0.11 |

improved size uniformity of the powder. Nova Scotia samples were notably inferior in yield, mat texture, and ease of mat isolation. Rehydration of the latter prior to processing provided improved yield. Attempts to use a Polytron™ for processing whole, dried, or powdered seaweed failed to provide any advantage over the blender, with VBrPO and mat yield decreasing an additional 20% and 56% respectively.

Moisture content aside, it might be significant that English harvest #2 yielded older plants that were further into the reproductive cycle than those from harvest #1. Extraction of 50g of the two materials under identical conditions showed that harvest #1 samples yielded an average of 61% more VBrPO (data not shown). However, a subsequent assessment of the impact of TPAS conditions on English *A. nodosum* was quite revealing. As $KP_i$ concentration was varied, VBrPO yield in the mat tracked separately from either mat mass or total activity.

Under conditions yielding the highest overall VBrPO levels in harvest #1 (Preps #16-20), a significant amount of activity was still associated with the pellet. A 0.1M reduction in $KP_i$ concentration lead to a moderate increase in mat mass, improved recovery of VBrPO activity in the mat, and a corresponding decrease in pellet bound enzyme. Of particular importance is that harvest #2 showed an optimal $KP_i$ concentration 0.1M lower than *A. nodosum* harvest #1, contrary to what would be expected if raw material moisture content was the sole contributing factor. The seaweed's stage of development therefore may be an important parameter with regards to the selection of TPAS conditions. Regardless, harvest to harvest variability is at present an unknown and therefore requires monitoring to maintain max-

imum yield. The optimum KP$_i$ concentration for extraction of samples from a given harvest appears to be constant (Table 9). The only deviation identified to date (Prep #32) was due to inefficient removal of PEG and KP$_i$ prior to lyophilization.

Table 9.

| Comparison of lyophilized mat yields using a constant TPAS salt concentration. | | | | |
|---|---|---|---|---|
| Prep # | Dry plant (g) | Wet mat (g) | Lyophilized mat (g) | Lyophilized mat/Dry plant (g/g) |
| 26 | 60 | 66 | 23 | 0.4 |
| 27 | 60 | 62 | 21 | 0.4 |
| 28 | 20 | 21 | 6.9 | 0.3 |
| 29 | 20 | 27 | 9.3 | 0.5 |
| 30 | 20 | 29 | 10 | 0.5 |
| 32 | 5 | 25 | 8.0 | 1.6 |
| 34 | 20 | 38 | 12 | 0.6 |
| 35 | 20 | 26 | 8.6 | 0.4 |
| 36 | 40 | 46.1 | 16 | 0.4 |
| 37 | 80 | 82 | 29 | 0.4 |
| 38 | 60 | 47 | 19 | 0.3 |

Another explanation for seasonal variability of VBrPO levels for *A. nodosum* from a given locality has been reported [Itoh, N., Sasaki, H., Ohsawa, N., Shibata, M.S., & Miura, J. (1996). Bromoperoxidase in *Corallina pilulifera* is regulated by its vanadate content. *Phytochemistry* 42(2), 277-281.] Changing vanadium levels and not *in vivo* VBrPO concentrations may be responsible, reflecting this enzyme's loose association with vanadate ions and re- emphasizing the importance of full VBrPO reactivation prior to assay. VBrPO stability in the mat after 6-57 days storage at ambient temperature was tested following rehydration (Table 10). The loss of activity was fairly consistent. Reactivation of VBrPO in the mat with 1mM ammonium vanadate for up to an hour proved of limited benefit. Parallel experiments using nonlyophilized mat that had simply been stored frozen showed a similar but less consistent pattern of inactivation (22% gain to 62% loss), with reactivation restoring as little as 4% of the lost activity

Table 10.

| Comparison of pre- and post-lyophilization VBrPO activity levels in mat using Phenol Red. | | | | |
|---|---|---|---|---|
| Prep# | Wet Mat (U/g) | Theoretical Lyoph. Mat (U/g) | Rehydrated Lyoph. Mat (U/g) | Activity Loss (%) |
| 12 | 1.7 | 4.6 | 3.1 | 32 |
| 16 | 1.4 | 3.8 | 2.8 | 43 |
| 18 | 3.0 | 8.8 | 4.1 | 54 |
| 20 | 2.8 | 8.7 | 3.5 | 60 |
| 22 | 4.7 | 13.6 | 4.2 | 69 |
| 23 | 5.6 | 16.9 | 6.6 | 61 |

During a subsequent search for conditions for VBrPO extraction from the mat, the presence of 2M Urea + 25mM tris pH 9.2 during reactivation was found to restore 75-150% of the pre-lyophilization VBrPO activity[36].

Although a direct comparison of mat verses traditional extract might seem unfair, the increase in purity realized by the early removal of troublesome contaminants actually makes the mat more comparable to the mid-process stream intermediate of a conventional process. Table 11 shows TPAS process efficiency upon scale-up and a projection of VBrPO yields in the mat. Expectations of 10,000U or ~167mg crude VBrPO per kilogram wet seaweed are not unrealistic for laboratory-scale production. In contrast, extrapolation of Wever's data suggests an initial extraction yield of only ~3000U, or 50mg crude VBrPO per kilo-

Table 11.

| TPAS Scale-up of harvest #2 under optimized conditions. | | | | |
|---|---|---|---|---|
| Prep # | Dry / Wet Plant Mass (g) | Wet Mat Mass (g) | Total Mat Activity (U) | Projected Yield / Kg Wet Seaweed (kU/kg) |
| 35 | 20/133 | 25.6 | 1413 | 10.6 |
| 36 | 40/267 | 46.1 | 2440 | 9.1 |
| 38 | 60/400 | 47 | 4040 | 10.1 |
| 37 | 80/533 | 81.7 | 6263 | 11.8 |

gram wet seaweed. Similarly, employing an optimized four-cycle 0.5L conventional extraction our lab could only be expected to release 3600U or 60mg VBrPO per kilogram wet seaweed.

Conventional purification procedures were deemed inefficient, wasteful, and potentially unsafe. Precautions against enzyme inhibition and chemical modification were minimal at best, but potential improvements were noted. Unoptimized extraction conditions necessitated multiple reextractions of the raw material. The resulting low quality extract required several lengthy, low resolution and low yield treatments to remove bulk contaminants. High resolution chromatography was pushed far down the production stream.

TPAS offered a surprisingly effective solution to the unique problems encountered by previous investigators. The advantages to early implementation of a chromatography step were quickly apparent.

Table 12.

| Summary of sample preparation and TPAS protocols. | |
|---|---|
| Step | Procedure |
| 1 | Wash / air dry seaweed. |
| 2 | Homogenization / TPAS |
| 3 | Centrifugation |
| 4 | Centrifugation (optional) |
| 5 | Lyophilization (optional) |

Reactive phenolics, viscous alginates, cell debris and most pigments were quickly removed while a concentrated VBrPO enriched fraction was isolated using a simple blend/centrifuge protocol. The reagents were inexpensive and prudent use was made of the entire plant. The instrumentation was simple, the process readily scaleable, and labor greatly reduced. Lyophilization of the mat produced a concentrated intermediate that was stable to extended ambient temperature storage, allowing our lab to stockpile material for succeeding purification studies.

A thorough investigation of relevant parameters was performed and key variables identified. PEG 8000 and 20,000 offered unique selectivity characteristics for VBrPO isolation from seaweed or contrived sources (e.g. fermentations). Conditions for phase stability were well defined. Reactivation requirements and the tendency for sample degradation were found to vary with sample type and storage conditions. Predrying the seaweed dramatically simplified raw material storage, handling, and homogenization. *A. nodosum* harvest-to-harvest variability in activity and TPAS performance was found to have multiple components, including vendor, moisture content, and possibly plant age. Importantly, performance was consistent within a given harvest.

A reference was found describing a TPAS purification scheme for VBrPO from an alternative species [Jordan, P. and Vilter, H. (1991). Extraction of proteins from material rich in anionic mucilages: Partition and fractionation of vanadate-dependent bromoperoxidases from the brown algae *Laminaria digitata* and *L. saccharina* in aqueous polymer two-phase systems. *Biochim. Biophys. Acta* 1073, 98-106.] The conditions employed were significantly different than those reported in the present invention, the investigators even mentioning the unsuitability of polymers larger than PEG 1550. The reference procedure was more consistent with a typical four step TPAS protocol producing a soluble VBrPO extract instead of a semi-solid plant fraction. Despite the successful separation of multiple VBrPO isoforms, the overall scheme proved quite elaborate and was ineffective when applied to *A. nodosum* [Butler, A. and Walker, J. V. (1993). Marine Haloperoxidases. *Chem. Rev.* 93, 1937-1944.]

The above experiments and examples are given to illustrate the scope and spirit of the present invention. These embodiments and examples will make apparent to those skilled in the art, other embodiments and examples. These other embodiments and examples are within the contemplation of the present invention; therefor, the instant invention should be limited only by the appended claims.

**Claims**

1. A method for purifying a haloperoxidase from a natural source comprising the steps of:

   i) homogenizing a sample containing the haloperoxidase, thereby producing an homogenate;
   ii) forming a mixture having a pH between pH 6.0 to pH 10.0, comprising

      a) the homogenate produced in step i)
      b) a salt, and
      c) a polymer having a molecular weight between about 6000 to about 20,000 daltons, wherein the concentration of the polymer in the mixture is between 7 to 20 percent by weight, the concentration of the salt is between 0.6 to 1.7 Molar, and wherein the mixture is capable of forming separate polymer and salt phases and an interphase zone between the polymer and salt phases; or

   iii) homogenizing a sample containing the haloperoxidase together with a salt, thereby producing an homogenate;
   iv) forming a mixture having a pH between pH 6.0 to pH 10.0, comprising

      d) the homogenate formed in step iii), and
      e) a polymer having a molecular weight between about 6000 to about 20,000 daltons, wherein the concentration of the polymer in the mixture is between 7 to 20 percent by weight, the concentration of the salt is between 0.6 to 1.7 Molar, and wherein the mixture is capable of forming separate polymer and salt phases and an interphase zone between the polymer and salt phases; or

   v) forming a mixture having a pH between pH 6.0 to pH 10.0 comprising a sample containing the haloperoxidase, a salt, and a polymer having a molecular weight between about 6000 to about 20,000 daltons, wherein the concentration of the polymer in the mixture is between 7 to 20 percent by weight, and the concentration of the salt is between 0.6 to 1.7 Molar,
   vi) homogenizing the mixture of step v), wherein the homogenized mixture is capable of forming separate polymer and salt phases and an interphase zone between the polymer and salt phases;
   vi) isolating the interphase zone;
   vii) solubilizing haloperoxidase that is present in the interphase zone; and
   viii) isolating the solubilized haloperoxidase.

2. The method of claim 1, wherein the mixture of step ii), step iv) or step vi) is centrifuged to hasten formation of the separate polymer and salt phases and the interphase zone.

3. The method of claim 1 or claim 2, wherein the natural source is seaweed, the pH of the mixture is between pH 6.0 to pH 9.0, the salt is a phosphateor sulphate salt, the concentration of the salt in the mixture is between 0.9 to 1.4 Molar, the polymer is polyethylene glycol, and the concentration of the polyethylene glycol is between 10 to 15 percent by weight.

4. The method of claim 1 or claim 2, wherein the haloperoxidase is vanadium bromoperoxidase from Ascophyllum nodosum, the pH of the mixture is between pH 8.0 to pH 8.5, the salt is sodium or potassium phosphate, the concentration of the salt in the mixture is between 0.9 to 1.2 Molar, the molecular weight of the polyethylene glycol is between about 8000 daltons to about 20,000 daltons, and the concentration of the polyethylene glycol is between 11 to 13 percent by weight.

5. The method of claim 4, wherein the vanadium bromoperoxidase is solubilized by forming a mixture having a pH between pH 7.5 to pH 10.0, comprising the isolated interphase zone and a urea or a guanidine salt, wherein the concentration of urea or guanidine salt in the mixture is at least about 1.6 Molar.

6. The method of claim 5, wherein the vanadium bromoperoxidase is isolated from the mixture comprising urea or guanidine salt, by centrifuging or filtering the mixture, thereby separating particulates and soluble vanadium bromoperoxidase.

7. A method for purifying a haloperoxidase from a fermentation or cell culture comprising the steps of:

i) forming a first mixture having a pH between pH 6.0 to pH 10.0, comprising,

a) a sample containing haloperoxidase,
b) a salt, and
c) a polymer having a molecular weight between about 6000 to about 20,000 daltons, wherein the concentration of the polymer in the first mixture is between 7 to 20 percent by weight, the concentration of the salt is between 0.6 to 1.7 Molar, and wherein the first mixture is capable of forming separate polymer and salt phases;

ii) isolating the polymer phase;
iii) forming a second mixture having a pH between pH 6.0 to pH 10.0, comprising

d) the polymer phase of step ii), and
e) a salt, wherein the concentration of the salt in the second mixture is between 0.6 to 0.9 Molar, and wherein the second mixture is capable of forming separate polymer and salt phases;

iv) isolating the salt phase of the second mixture, the salt phase comprising haloperoxidase.

8. A method for further purifying a haloperoxidase prepared according to claim 1, comprising the steps of:

i) forming a first mixture having a pH between pH 6.0 to pH 10.0, comprising,

a) a sample containing haloperoxidase,
b) a salt, and
c) a polymer having a molecular weight between about 6000 to about 20,000 daltons, wherein the concentration of the polymer in the first mixture is between 7 to 20 percent by weight, the concentration of the salt is between 0.6 to 1.7 Molar, and wherein the first mixture is capable of forming separate polymer and salt phases;

ii) isolating the polymer phase;
iii) forming a second mixture having a pH between pH 6.0 to pH 10.0, comprising

d) the polymer phase of step ii), and
e) a salt, wherein the concentration of the salt in the second mixture is between 0.6 to 0.9 Molar, and wherein the second mixture is capable of forming separate polymer and salt phases;

iv) isolating the salt phase of the second mixture, the salt phase comprising haloperoxidase.

9. The method of claim 7 or claim 8, wherein the haloperoxidase is vanadium bromoperoxidase, the salt in the first and second mixtures is sodium or potassium phosphate or sulfate, the concentration of the salt in the first mixture is between 0.9 to 1.7 Molar.

10. A composition or dry analytical element comprising a haloperoxidase purified according to any one of claims 1 to 9.